(19) Europäisches Patentamt
European Patent Office
Office européen des brevets

(11) **EP 3 105 571 B1**

(12) **EUROPÄISCHE PATENTSCHRIFT**

(45) Veröffentlichungstag und Bekanntmachung des Hinweises auf die Patenterteilung:
**25.07.2018 Patentblatt 2018/30**

(51) Int Cl.:
*G01N 27/12* (2006.01)    *G01N 27/28* (2006.01)

(21) Anmeldenummer: **15707890.8**

(22) Anmeldetag: **11.02.2015**

(86) Internationale Anmeldenummer:
**PCT/EP2015/052891**

(87) Internationale Veröffentlichungsnummer:
**WO 2015/121312 (20.08.2015 Gazette 2015/33)**

(54) **VERFAHREN UND SENSORSYSTEM ZUR MESSUNG DER KONZENTRATION VON GASEN**

METHOD AND SENSOR SYSTEM FOR MEASURING GAS CONCENTRATIONS

PROCÉDÉ ET SYSTÈME DE CAPTEUR DE MESURE DE LA CONCENTRATION DE GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.02.2014 DE 102014101657**

(43) Veröffentlichungstag der Anmeldung:
**21.12.2016 Patentblatt 2016/51**

(73) Patentinhaber: **ams Sensors Germany GmbH 07745 Jena (DE)**

(72) Erfinder: **GRAUNKE, Thorsten 72076 Tübingen (DE)**

(74) Vertreter: **Epping - Hermann - Fischer Patentanwaltsgesellschaft mbH Schloßschmidstraße 5 80639 München (DE)**

(56) Entgegenhaltungen:
**DE-A1-102007 040 726**    **JP-A- 2005 134 311**
**JP-A- 2007 024 508**    **JP-A- 2007 271 441**
**US-A1- 2009 084 160**

- **CAVICCHI R E ET AL: "Fast temperature programmed sensing for micro-hotplate gas sensors", IEEE ELECTRON DEVICE LETTERS, IEEE SERVICE CENTER, NEW YORK, NY, US, Bd. 16, Nr. 6, 1. Juni 1995 (1995-06-01), Seiten 286-288, XP011430440, ISSN: 0741-3106, DOI: 10.1109/55.790737**

EP 3 105 571 B1

**Beschreibung**

Die vorliegende Patentanmeldung betrifft ein Verfahren und ein Sensorsystem zur Messung der Konzentration mindestens eines Gases aus einer Gasprobe.

Sensorsysteme zur Detektion von Gasen aus einer Gasprobe sind in vielfältigen Ausgestaltungen bereits bekannt. Die Genauigkeit der Konzentrationsmessung eines Gases hängt bei vielen Sensoren stark von der Reaktivität des zu detektierenden Gases ab.

Darüber hinaus wird die Konzentrationsbestimmung eines Gases durch das Vorhandensein anderer Gase beeinflusst. Ein von einem einzelnen Gas stammendes Sensorsignal kann im Rauschen der Sensorsignale konkurrierender Gase untergehen, d.h. das Sensorsignal des zu detektierenden Gases wird durch die Sensorsignale der konkurrierenden Gase überdeckt bzw. überlagert. Metall-Oxid-Sensoren weisen beispielsweise für einzelne Gase in bestimmten Temperaturbereichen hohe Signalstärken auf, zeigen aber bei Gasproben aus mehreren Gasen eine Überlagerung der Signale. Hierdurch wird die Selektivität bekannter Sensoreinrichtungen mit solchen Sensoren beschränkt.

Dokument US 2009/0084160 A1 beschreibt ein Gasmessbauelement, das einen Sensor und ein Sensorgehäuse mit einem Sensorhohlraum umfasst. Das Sensorgehäuse umfasst Seitenwände, einen geschlossenen Boden und ein offenes oberes Ende, das einen Filter oder ein Siebelement aufweist. Ein Heizelement des Sensors wird verwendet, um die Temperatur eines Sensorelements auf eine vorgegebene Temperatur anzuheben.

Publikation R. E. Cavicchi et al., "Fast Temperature Programmed Sensing for Micro-Hotplate Gas Sensors", IEEE Electron Device Letters, Band 16, Nr. 6, Juni 1995, Seiten 286 bis 288, erläutert einen Sensor mit einer miniaturisierten Heizplatte. Der Sensor befindet sich im freien Raum.

Dokument JP 2005-134311 A gibt einen Halbleitergassensor an. Der Gassensor umfasst einen Widerstand. Eine Temperatur des Widerstands wird auf einen ersten Temperaturwert für eine vorgegebene Zeit gehalten. Anschließend wird die Temperatur des Widerstands vom ersten auf einen zweiten Temperaturwert geändert und der Widerstand beim zweiten Temperaturwert für eine vorgegebene Zeit gehalten. Die Gasdetektion basiert auf den Werten des Widerstands bei den verschiedenen Messschritten.

Der Erfindung liegt daher die Aufgabe zugrunde, ein Verfahren und ein Sensorsystem zur Bestimmung der Konzentration eines Gases mit höherer Genauigkeit und Selektivität bereitzustellen.

Diese Aufgabe wird erfindungsgemäß gelöst durch ein Verfahren und ein Sensorsystem gemäß den unabhängigen Ansprüchen. Weiterbildungen und Ausgestaltungen sind jeweils Gegenstand der abhängigen Ansprüche.

In einer Ausführungsform ist ein Verfahren eingerichtet zur Messung der Konzentration mindestens eines Gases aus einer Gasprobe mit einem Sensorsystem, das einen Messbereich mit mindestens einem Gassensor, vorzugweise einem Metall-Oxid-Sensor, aufweist, wobei der Messbereich eine Diffusionsöffnung aufweist, die durch eine gasdurchlässige Struktur verschlossen ist, dadurch gekennzeichnet, dass der Messbereich zunächst aufgeheizt wird, dann die Heizung abgeschaltet und die Widerstandsänderung des mindestens einen Gassensors gemessen wird.

Durch das Heizen des Messbereichs wird die Gaskonzentration bei konstantem Druck und Volumen im Messbereich verringert. Nach Abschalten der Heizung erhöht sich die Konzentration, was zu einer Widerstandsänderung in dem mindestens einen Gassensor führt. Dadurch kann die Konzentration der im Messbereich befindlichen Gase gemessen werden. Durch die künstlich mittels Diffusion erzeugte Konzentrationsänderung kann die Sensitivität des Sensorsystems erheblich gesteigert werden.

Durch die Bestimmung des Widerstandes des Gassensors bei zwei unterschiedlichen Konzentrationen des Gases im Messbereich wird die Genauigkeit der Messung der Konzentration des Gases im Raum außerhalb des Sensorsystems erhöht.

Alternativ wird anstelle einem Abschalten der Heizung die Heizleistung auf einen von 0 verschiedenen Wert verringert.

Sowohl durch das Abschalten der Heizung wie auch durch das Verringern der Heizleistung sinkt die Temperatur der Gasprobe im Messbereich gegenüber der Phase, in welcher der Messbereich aufgeheizt wird. Zur Bestimmung der Widerstandsänderung des Gassensors werden mindestens ein Widerstandswert des Gassensors bei einer hohen Temperatur des Messbereichs und mindestens ein Widerstandswert des Gassensors bei einer niedrigen Temperatur des Messbereichs gemessen.

Zusätzlich kann auch die Selektivität des Sensorsystems gesteigert werden. Neben einer auf das Zielgas abgestimmten Auswahl der sensiven Schicht des mindestens einen Gassensors lässt sich die Selektivität des Sensorsystems durch eine Sensorheizeinrichtung für den mindestens einen Gassensor und/oder durch die Heizung des Messbereichs einstellen. Die Signalstärken von Metall-Oxid-Sensoren sind bei gleichbleibender Konzentration für verschiedene Gase unterschiedlich. Die Signalstärken können somit über eine Temperaturmodulation der Sensorheizeinrichtung für den Gassensor und/oder der Heizung des Messbereichs verändert werden.

Das Verfahren lässt sich in den unterschiedlichsten Anwendungsgebieten einsetzen. So kann die Gasprobe ein Einzelgas, ein Gasgemisch und/oder ein Aerosol sein.

Die Sensitivität und Selektivität des Sensorsystems lässt sich für bestimmte Anwendungsfälle zusätzlich dadurch erhöhen, dass die Gasprobe durch mindestens eine vorzugsweise beheizbare Katalysatoranordnung hindurchströmt und/oder diffundiert, bevor sie das Sensorsystem oder den Messbereich des Sensorsystems erreicht. Die Katalysatoranordnung kann dabei in die gasdurchlässige Struktur an der Diffusionsöffnung des Messbereichs integriert sein. Die Gase der Gasprobe werden durch die Katalysatoranordnung zumindest teilweise in andere Gase umgewandelt, die von dem Gassensor entweder leichter zu detektieren oder überhaupt nicht zu detektieren sind und daher das Messergebnis nicht beeinflussen, um ein genaueres Messergebnis zu erhalten.

In einer Ausführungsform ist ein Sensorsystem eingerichtet zur Messung der Konzentration mindestens eines Gases aus einer Gasprobe mit einem Messbereich, in dem mindestens ein Gassensor, vorzugsweise ein Metall-Oxid-Sensor, angeordnet ist und der eine Diffusionsöffnung aufweist, die durch eine gasdurchlässige Struktur verschlossen ist und die dadurch gekennzeichnet ist, dass der Messbereich mit einer regelbaren Heizeinrichtung für den Messbereich versehen ist.

Das Sensorsystem kann auch als Sensoreinrichtung bezeichnet werden.

Mit diesem Sensorsystem lässt sich das Verfahren zur Bestimmung der Konzentration eines Gases mit hoher Genauigkeit durchführen. Durch eine entsprechende Regelung der Heizeinrichtung kann außerdem die Selektivität der Sensoranordnung erhöht werden.

In einer Ausführungsform ist das Sensorsystem eingerichtet, dass der Messbereich zunächst durch die Heizeinrichtung aufgeheizt wird und dann die Heizung abgeschaltet wird oder die Heizleistung auf einen von 0 Watt verschiedenen Wert verringert wird. Weiter kann das Sensorsystem eingerichtet sein, dass eine Widerstandsänderung des mindestens einen Gassensors gemessen wird.

Bei einer Ausgestaltung des Sensorsystems ist die die Diffusionsöffnung des Messbereichs verschließende Struktur durch die Heizeinrichtung beheizbar.

Zusätzlich kann auch der mindestens eine Gassensor beheizbar sein, um ihn optimal an das zu detektierende Gas anpassen zu können und somit die Selektivität des Sensorsystems zu steigern.

Die die Diffusionsöffnung des Messbereichs verschließende Struktur kann ein gasdurchlässiges Gitter, ein Netz, ein poröser Festkörper, ein Schwamm oder eine Membran sein. Alternativ kann ein modulierter Wärmestrahler als Heizeinrichtung oberhalb des Gassensors eingesetzt werden, der so in der Diffusionsöffnung aufgehängt werden kann, dass das Gas an ihm vorbeiströmen kann.

Die Temperatur der Gasprobe im Messbereich kann somit moduliert werden. Durch die Modulation der Temperatur der Gasprobe wird die Konzentration der verschiedenen Gase in der Gasprobe moduliert.

In einer Ausführungsform ist die Konzentration eines Gases der Gasprobe im Messbereich in einer ersten Phase, während der der Messbereich aufgeheizt wird, verschieden von der Konzentration des Gases der Gasprobe im Messbereich in einer zweiten Phase, während der die Heizung abgeschaltet oder die Heizleistung auf einen von 0 verschiedenen Wert verringert ist. Im Allgemeinen ist die Konzentration in der zweiten Phase höher als in der ersten Phase.

Der Messbereich kann auch als Gasraum, Messkammer oder Sensorkammer bezeichnet werden. Der Messbereich wird von der Gasprobe gefüllt. Der Messbereich kann frei von Festkörpern sein.

In einer Ausführungsform weist die gasdurchlässige Struktur und der mindestens eine Gassensor einen Abstand voneinander auf. Dieser freie Zwischenraum wird als Messbereich bezeichnet. Durch diesen Abstand entsteht das Volumen des Messbereiches.

Die Diffusionsöffnung ist somit der Einlass des Messbereichs.

In einer Ausführungsform erfolgt ein Austausch des Gases/der Luft im Messbereich mit dem Gas/der Luft außerhalb des Sensorsystems ausschließlich durch die Diffusionsöffnung.

In einer Ausführungsform weist der Messbereich eine einzige Diffusionsöffnung auf.

In einer Ausführungsform weist der Messbereich mindestens eine Diffusionsöffnung auf. Ein Austausch des Gases/der Luft im Messbereich mit dem Gas/der Luft außerhalb des Sensorsystems erfolgt ausschließlich durch die mindestens eine Diffusionsöffnung.

In einer Ausführungsform ist das Sensorsystem als ein Mikrosystem, englisch micro-electro-mechanical system, realisiert.

Die Erfindung wird im Folgenden an mehreren Ausführungsbeispielen anhand der Figuren näher erläutert. Funktions- beziehungsweise wirkungsgleiche Komponenten, Schichten oder Strukturen tragen gleiche Bezugszeichen. Insoweit sich Komponenten, Schichten oder Strukturen in ihrer Funktion entsprechen, wird deren Beschreibung nicht in jeder der folgenden Figuren wiederholt. Es zeigen:

Figur 1              eine beispielhafte Ausführungsform eines Sensorsystems,

Figuren 2A und 2B    eine weitere beispielhafte Ausführungsform des Sensorsystems,

Figuren 3 und 4      weitere beispielhafte Ausführungsformen des Sensorsystems,

Figur 5          eine beispielhafte Ausführungsform eines Schaltbildes des Sensorsystems und

Figur 6          eine beispielhafte Ausführungsform der Signale im Sensorsystem.

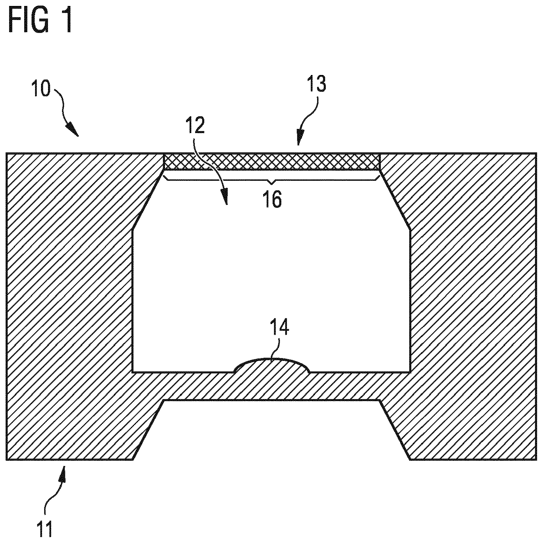

Figur 1 zeigt eine beispielhafte Ausführungsform eines Sensorsystems 10. Nachfolgend wird ein Ausführungsbeispiel des Sensorsystems 10 mit Bezug auf die Zeichnung, die einen Querschnitt durch das Sensorsystem 10 zeigt, näher beschrieben.

Das Sensorsystem 10 weist ein gasdichtes Gehäuse 11 auf, das einen Messbereich 12 umschließt. Eine Diffusionsöffnung 16 des Messbereichs 12 ist durch eine gasdurchlässige Struktur 13 verschlossen. Durch diese kann Gas aus der Umgebung in den Messbereich 12 und zu einem Gassensor, hier einem Metall-Oxid-Sensor 14, strömen und/oder diffundieren. Im dargestellten Beispiel ist die gasdurchlässige Struktur 13 durch eine nicht näher dargestellte Heizeinrichtung mit einer Regeleinrichtung beheizbar. Wird die gasdurchlässige Struktur 13 aufgeheizt, so erwärmt sich dadurch auch der Innenraum des Messbereichs 12, was zu einer Verringerung der Gaskonzentration im Messbereich 12 führt. Wird anschließend die Beheizung der gasdurchlässigen Struktur 13 gestoppt, diffundiert durch die Abkühlung des Messbereichs 12 mehr Gas durch die gasdurchlässige Struktur 13 in den Messbereich 12, was zu einer Widerstandsänderung des Metall-Oxid-Sensors 14 führt. Diese Widerstandsänderung wird von einer hier nicht dargestellten Auswerteeinrichtung erfasst. Sie ist ein Maß für die Gaskonzentration im Messbereich 12 und damit auch in der Umgebung, sobald der Messbereich 12 und die Umgebung die gleiche Temperatur erreicht haben. Auch der Metall-Oxid-Sensor 14 selbst kann beheizbar sein. Dadurch lässt er sich optimal an verschiedene Gase anpassen. Zudem kann die Selektivität des Sensorsystems 10 durch eine aufeinander abgestimmte Modulation der Temperatur der Heizeinrichtungen für die gasdurchlässige Struktur 13 und für den Gassensor 14 erhöht werden.

Die gasdurchlässige Struktur 13 kann eine Membran, ein poröser Festkörper, ein Schwamm oder ein Gitter sein. Außerdem kann die gasdurchlässige Struktur 13 aus einem katalytisch wirkenden Material gefertigt sein. Weiter ist es möglich und zweckmäßig, im Messbereich 12 einen hier nicht dargestellten Temperatursensor und ggf. einen Feuchtesensor anzuordnen, der ebenfalls mit der Auswerteeinrichtung für die Sensorsignale und der Regeleinrichtung der Heizeinrichtungen für den Messbereich 12 und den Gassensor 14 verbunden ist.

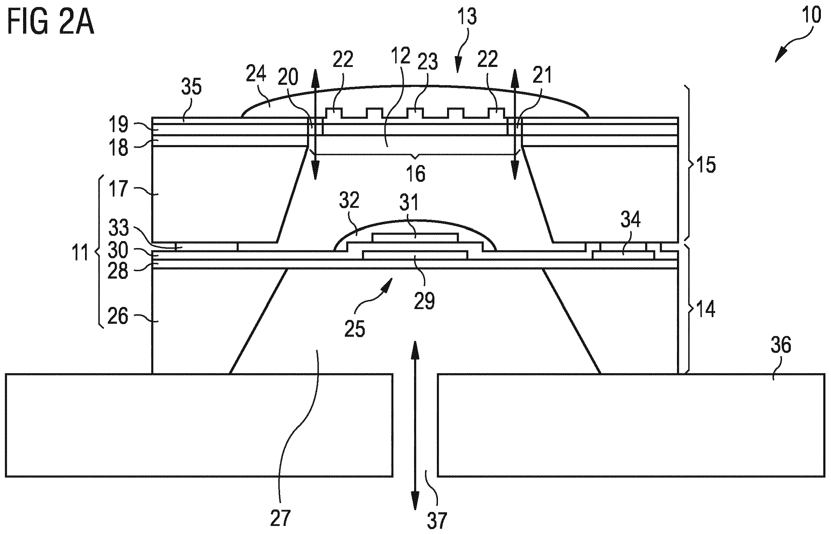

Figur 2A zeigt eine weitere beispielhafte Ausführungsform des Sensorsystems 10 im Querschnitt, welches eine Weiterbildung der in Figur 1 gezeigten Ausführungsform ist. Das Sensorsystem 10 umfasst einen Messbereichs-Halbleiterkörper 15, der die Diffusionsöffnung 16 aufweist. Der Messbereichs-Halbleiterkörper 15 ist als mikromechanisches Bauteil implementiert. Die Diffusionsöffnung 16 ist durch die gasdurchlässige Struktur 13 geschlossen. Der Messbereichs-Halbleiterkörper 15 ist als Siliziumbauteil realisiert. Der Messbereichs-Halbleiterkörper 15 ist aus einem Silicon-on-Insulator Wafer, abgekürzt SOI-Wafer, hergestellt. Die gasdurchlässige Struktur 13 weist eine Isolatorschicht 35 auf. Die Isolatorschicht 35 kann aus Siliziumnitrid, insbesondere $Si_3N_4$, sein.

Weiter umfasst die gasdurchlässige Struktur 13 eine Trägerschicht 19. Die Trägerschicht 19 kann eine Silizium-Schicht, insbesondere aus einkristallinem Silizium, sein. Die gasdurchlässige Struktur 13 ist auf einem Rahmen 17 des Messbereichs-Halbleiterkörpers 15 angeordnet. Auf dem Rahmen 17 befindet sich eine isolierende Schicht 18. Die isolierende Schicht 18 kann aus Siliziumoxid, insbesondere $SiO_2$, sein. Dabei ist die isolierende Schicht 18 zwischen der Trägerschicht 19 und dem Rahmen 17 angeordnet. Die Isolatorschicht 35 ist auf der Trägerschicht 19 angeordnet. Der Messbereich 12 ist vor allem eine mit Methoden der Mikromechanik geätzte Ausnehmung im Messbereichs-Halbleiterkörper 16.

Der Schichtaufbau, umfassend die Isolatorschicht 35 und die Trägerschicht 19, weist mindestens eine Öffnung 20, 21 auf. Gas kann durch die mindestens eine Öffnung 20, 21 vom Außenraum des Sensorsystems 10 in den Messbereich 12 diffundieren. Die zwei Öffnungen 20, 21 sind als durchgehende Löcher realisiert. Weiter umfasst die gasdurchlässige Struktur 13 eine Heizeinrichtung 22. Die Heizeinrichtung 22 ist als Heizwiderstand realisiert. Die Heizeinrichtung 22 ist auf der Isolatorschicht 35 angeordnet. Die Heizeinrichtung 22 ist in die gasdurchlässige Struktur 13 integriert.

Die gasdurchlässige Struktur 13 umfasst einen Temperatursensor 23. Der Temperatursensor 23 kann als Temperaturmesswiederstand ausgebildet sein. Die Heizeinrichtung 22 und der Temperatursensor 23 können aus einem Metalldünnfilm, insbesondere aus Platin oder Nickel, hergestellt sein. Der Temperatursensor 23 ist neben der Heizeinrichtung 22 auf der Isolatorschicht 35 angeordnet. Der Temperatursensor 23 ist in der Mitte der gasdurchlässigen Struktur 13 lokalisiert. Die Heizeinrichtung 22 ist um den Temperatursensor 23 herum angeordnet. Der Messbereichs-Halbleiterkörper 15 ist somit als Infrarotstrahler realisiert.

Ferner umfasst die gasdurchlässige Struktur 13 eine gaspermeable Abdeckschicht 24. Die gaspermeable Abdeckschicht 24 bedeckt die mindestens eine Öffnung 20, 21 in dem Schichtaufbau, umfassend die Isolatorschicht 35 und die Trägerschicht 19. Die gaspermeable Abdeckschicht 24 kann porös sein. Die gaspermeable Abdeckschicht 24 kann als gesinterte Schicht realisiert sein. Beispielsweise kann die gaspermeable Abdeckschicht 24 als gesinterte Keramik hergestellt sein. Die Keramik kann beispielsweise hauptsächlich Aluminiumoxid, Zinnoxid oder Siliciumcarbid, insbesondere $Al_2O_3$ oder $SnO_2$ oder SiC, aufweisen.

Die gaspermeable Abdeckschicht 24 kann auch als Katalysatorschicht oder Katalysatoranordnung realisiert sein. Dazu weist sie beispielsweise Palladium und/oder Platin und/oder Gold als Material auf. Die gesinterte Keramikschicht kann somit Palladium- und/oder Platin- und/oder Goldanteile zusätzlich zum Grundmaterial wie etwa Aluminiumoxid, Zinnoxid oder Siliciumcarbid aufweisen. Die gasdurchlässige Struktur 13 kann somit auch die Katalysatoranordnung umfassen.

Die gasdurchlässige Struktur 13 weist somit ein gasdurchlässiges Gitter und einen porösen Festkörper auf. Das gasdurchlässige Gitter ist durch den Schichtaufbau, der die Isolatorschicht 35 und die Trägerschicht 19 umfasst, und die mindestens eine Öffnung 20, 21 in dem Schichtaufbau ausgebildet. Der poröse Festkörper kann durch die gaspermeable Abdeckschicht 24 realisiert sein.

Weiter umfasst das Sensorsystem 10 den Gassensor 14. Der Gassensor 14 ist als Metall-Oxid-Sensor, auch Metall-Oxid-Halbleiter Gassensor genannt, realisiert. Der Gassensor 14 ist als mikromechanisches Bauteil implementiert. Der Gassensor 14 umfasst einen Sensorrahmen 26. Der Gassensor 14 weist eine Sensormembran 25 auf. Die Sensormembran 25 überspannt eine Ausnehmung 27 des Gassensors 14. Die Ausnehmung 27 befindet sich zwischen dem Sensorrahmen 26. Die Ausnehmung 27 ist mit Methoden der Mikromechanik geätzt. Die Sensormembran 25 ist nicht gasdurchlässig. Die Sensormembran 25 weist eine erste Isolatorschicht 28 auf. Die erste Isolatorschicht 28 kann als Siliziumnitrid-Schicht, insbesondere $Si_3N_4$-Schicht, hergestellt sein. Auf der ersten Isolatorschicht 28 ist über der Ausnehmung 27 eine Sensorheizeinrichtung 29 des Gassensors 14 angeordnet. Auf der Sensorheizeinrichtung 29 ist eine zweite Isolatorschicht 30 angeordnet. Die zweite Isolatorschicht 30 kann als Siliziumnitrid-Schicht, insbesondere $Si_3N_4$-Schicht, abgeschieden sein.

Der Gassensor 14 umfasst eine Elektrodenanordnung 31. Weiter weist der Gassensor 14 eine sensitive Schicht 32 auf. Auf der zweiten Isolatorschicht 30 befindet sich die Elektrodenanordnung 31. Die Elektrodenanordnung 31 kann als Interdigital-Elektrodenanordnung realisiert sein. Auf der Elektrodenanordnung 31 ist die sensitive Schicht 32 abgeschieden. Ist der Gassensor 14 als Metall-Oxid-Sensor realisiert, so ist die sensitive Schicht 32 ein Metalloxid. Das Metalloxid kann beispielsweise Zinnoxid, Zinkoxid, Galliumoxid oder Wolframoxid, insbesondere $SnO_2$, $ZnO$, $Ga_2O_3$ oder $WO_3$ sein. Das Metalloxid kann mit einem Edelmetall wie etwa Palladium oder Platin imprägniert sein.

Das Metalloxid kann als Keramik hergestellt sein. Die Keramik kann gesintert sein und aus Zinnoxid, Zinkoxid, Galliumoxid oder Wolframoxid bestehen. Der Gassensor 14 kann somit als Dickschichtsensor realisiert sein. Die sensitive Schicht 32 ist eine poröse Schicht. Die Schichtdicke der sensitiven Schicht 32 liegt typischerweise im Mikrometerbereich.

Die sensitive Schicht 32 kann zum Beispiel mittels Tropfen-Technik, Aerosol-Technik, Stößel-Technik oder Siebdruck Verfahren aufgebracht werden. Bei der Tropfen-Technik wird ein Tropfen des Ausgangsmaterials der sensitiven Schicht 32 auf die Elektrodenanordnung 31 aufgegeben. Der Tropfen wird mittels einer Nadel aufgebracht. Bei der Aerosol-Technik wird ein Aerosol aus dem Ausgangsmaterial etwa durch Verdampfen erzeugt. Das Aerosol schlägt sich auf dem Untergrund - wie der Elektrodenanordnung 31 und der zweiten Isolatorschicht 30 - nieder; die Schichtdicke der sensitiven Schicht 32 lässt sich dabei durch eine Öffnungszeit eines Shutters einstellen. In der Stößel-Technik wird mit Hilfe eines Stößels oder Stempels das Ausgangsmaterial aufgenommen und auf die Elektrodenanordnung 31 aufgebracht. Bei diesen unterschiedlichen Verfahren wird das Ausgangsmaterial getempert oder einem Annealing-Prozess unterzogen, so dass die als Keramik ausgebildete sensitive Schicht 32 entsteht.

Alternativ kann der Gassensor 14 als Dünnfilmsensor implementiert sein. Die sensitive Schicht 32 ist eine kompakte Schicht. Die Schichtdicke der sensitiven Schicht 32 liegt typischerweise im Nanometerbereich. Eine dünne sensitive Schicht 32 beispielsweise aus Zinnoxid, Zinkoxid, Galliumoxid oder Wolframoxid kann durch Sputtern oder Aufdampfen hergestellt sein.

Der Rahmen 26 und der Sensorrahmen 17 bilden das Gehäuse 11 des Sensorsystems 10. Der Messbereichs-Halbleiterkörper 15 und der Gassensor 14 sind über eine geeignete Verbindungstechnik miteinander verbunden. Dazu können eine Verbindungsschicht 33 zwischen dem Gassensor 14 und dem Messbereichs-Halbleiterkörper 15 angeordnet sein. Die Verbindung ist in Figur 2A nur angedeutet. Zur Verbindung kann beispielsweise ein temperaturresistenter Kleber wie etwa ein Zementkleber, ein Lot, eine eutektische Verbindung, ein Silicon Fusion Bonding oder eine anodische Verbindung eingesetzt werden. Weiter weist der Gassensor 14 mindestens eine Kontaktfläche 34, auch Bondpad genannt, auf. Eine elektrische leitende Verbindung zum Bondpad 34 von außen kann beispielsweise vor dem Aufsetzen des Messbereichs-Halbleiterkörpers 15 auf den Gassensor 14 realisiert werden.

Weiter weist das Sensorsystem 10 einen Träger 36 auf. Der Träger 36 kann als ein Keramikträger, eine Leiterplatte, englisch: Printed Circuit Board, oder als ein Sockel realisiert sein. Beispielsweise kann der Sockel ein Transistor Outline Sockel, abgekürzt TO-Sockel, sein. Der Träger 36 weist eine Trägeröffnung 37 auf, die zur Ausnehmung 27 führt. Durch die Trägeröffnung 37 kann Luft/Gas aus der Ausnehmung 27 ausströmen. Somit verhindert die Trägeröffnung 37 im Träger 36, dass ein Überdruck in der Ausnehmung 27 entsteht, der beispielsweise zur Zerstörung der Sensormembran 25 führen kann. Die Trägeröffnung 37 kann als Bohrung realisiert sein. Die Trägeröffnung 37 dient zum Druckausgleich zwischen der Ausnehmung 27 und der Umgebung. Der Sensorrahmen 26 ist über eine nicht gezeigte Verbindungstechnik mit dem Träger 36 verbunden.

Der Sensorheizeinrichtung 29 wird über zwei Bondpads 34 elektrische Energie zugeführt. Die Sensorheizeinrichtung 29 heizt die Sensormembran 25 und damit die sensitive Schicht 32 auf. Die Widerstandsänderung in der sensitiven Schicht 32 wird mittels der Elektrodenanordnung 31 gemessen. Ein Sensorsignal S3 ist an zwei weiteren Bondpads abgreifbar. Von den insgesamt vier Bondpads ist beispielhaft das Bondpad 34 gezeigt.

Die Heizeinrichtung 22 heizt den Schichtaufbau, umfassend die Isolatorschicht 35 und die Trägerschicht 19, sowie die gaspermeable Abdeckschicht 24 auf. Somit wird das Volumen des Messbereichs 12 aufgeheizt. Es wird dabei die Luft oder das Gas, das sich im Messbereich 12 befindet, durch die Heizeinrichtung 22 aufgeheizt. Der Temperatursensor 23 dient zum Messen der Temperatur der gasdurchlässigen Struktur 13. Der Messbereich 12 wird vor allem durch die Ausnehmung im Rahmen 17 realisiert. Der Messbereich 12 ist der von der Sensormembran 25, dem Rahmen 17 und der gasdurchlässigen Struktur 13 umschlossene Raum. Die Heizeinrichtung 22 und die Sensorheizeinrichtung 29 führen zu einem Aufheizen der Luft/des Gases im Messbereich 12. Ein durch das Aufheizen entstehender Überdruck wird durch die mindestens eine Öffnung 20, 21 abgebaut. Die Konzentration eines Gases ist die Anzahl der Moleküle des Gases pro Volumeneinheit. Die Konzentration des Gases nimmt durch das Heizen des Messbereichs 12 ab.

Durch das Abschalten beispielsweise der Heizeinrichtung 22 sinkt die Temperatur der Luft/des Gases im Messbereich 12, sodass Gas/Luft durch die gaspermeable Abdeckschicht 24 in den Messbereich 12 eindiffundiert. Die Konzentration des Gases nimmt somit beim Absinken der Temperatur zu.

Durch katalytisch wirkende Bestandteile der gaspermeablen Abdeckschicht 24 verändert sich die in dem Messbereich 12 einströmende Luft/Gas gegenüber der Luft/Gas, welche sich außerhalb des Sensorsystems 10 befindet. Durch die katalytisch wirkenden Bestandteile der gaspermeablen Abdeckschicht 24 kann beispielsweise Kohlenmonoxid in Kohlendioxid umgewandelt werden. Je nach verwendetem Katalysatormaterial und nach zu detektierendem Gas werden unterschiedliche Temperaturen in der gasdurchlässigen Struktur 13 eingestellt. In einem Beispiel kann die Temperatur in der gasdurchlässigen Struktur 350 bis 400 °C betragen.

In einer alternativen, nicht gezeigten Ausführungsform dient die Heizeinrichtung 22 gleichzeitig auch als Temperatursensor. Dabei kann der Temperatursensor 23 weggelassen werden.

In einer alternativen, nicht gezeigten Ausführungsform weist die gaspermeable Abdeckschicht 24 keine katalytisch wirksamen Bestandteile auf.

In einer alternativen, nicht gezeigten Ausführungsform kann die gaspermeable Abdeckschicht 24 entfallen. Da die Abmessungen der Öffnungen 20, 21 sehr klein gehalten sind, erfolgt der Gasaustausch des Messbereichs 12 mit dem Raum außerhalb des Sensorsystems 10 ausschließlich durch Diffusion.

In einer alternativen, nicht gezeigten Ausführungsform kann die Trägerschicht 19 entfallen. Die gasdurchlässige Struktur 13 ist damit frei von einkristallinem Silizium.

In einer alternativen, nicht gezeigten Ausführungsform ist der Gassensor 14 als Wärmetönungssensor realisiert. Ein Wärmetönungssensor weist keine Sensorelektroden 31 auf. Ist der Gassensor 14 als Wärmetönungssensor realisiert, so weist er den Sensorrahmen 26, die Sensormembran 25, die Sensorheizeinrichtung 29 und die sensitive Schicht 32 auf. Die sensitive Schicht 32 ist als katalytisch aktive Schicht realisiert. Die katalytisch aktive Schicht weist beispielsweise ein Edelmetall, wie Platin oder Palladium oder Metalloxide, wie beispielsweise Manganoxid oder Kupfer(II)Oxid auf. Wird Gas im Messbereich 12 an der sensitiven Schicht 32 umgesetzt, so führt die Reaktion zu einer Erhöhung der Temperatur der Sensormembran 25. Die Temperaturerhöhung kann mittels eines zusätzlichen Temperatursensors oder mittels Bestimmung des Widerstands der Sensorheizeinrichtung 29 bestimmt werden.

In einer alternativen, nicht gezeigten Ausführungsform ist der Gassensor 14 als Wärmeleitfähigkeitssensor realisiert. Dabei misst der Gassensor 14 die Wärmeleitfähigkeit zwischen der Sensormembran 25 und der gasdurchlässigen Struktur 13. Strömt Gas mit einer hohen Wärmeleitfähigkeit in den Messbereich 12 ein, so erhöht sich die Wärmeleitung zwischen einer heißen Oberfläche und einer kalten Oberfläche. Beispielsweise kann die Heizeinrichtung 22 eingeschaltet sein und die Sensorheizeinrichtung 29 ausgeschaltet sein. Somit wird das Aufheizen der Sensormembran 25 durch ein wärmeleitendes Gas mit der gasdurchlässigen Struktur 13 als Wärmequelle gemessen. Der Anstieg der Temperatur der Sensormembran 25 ist umso stärker, je besser wärmeleitend das Gas im Messbereich 12 ist.

Alternativ wird die Temperaturänderung der gasdurchlässigen Struktur 13 gemessen. Bei gleichbleibender Heizleistung der Heizeinrichtung 22 erhöht sich die Temperatur der gasdurchlässigen Struktur 13 bei einem wenig wärmeleitenden Gas im Messbereich 12 mehr als bei einem stärker wärmeleitenden Gas.

Alternativ wird die Sensormembran 25 mittels der Sensorheizeinrichtung 29 aufgeheizt, die Heizeinrichtung 23 deaktiviert und die Wärmeübertragung von der Sensormembran 25 zur gasdurchlässigen Struktur 13 gemessen. Auch hier kann entweder die Änderung der Temperatur der Sensormembran 25 oder der gasdurchlässigen Struktur 13 gemessen werden.

Der Gassensor 14 kann somit als chemischer Gassensor - wie etwa ein Metall-Oxid-Sensor - als chemisch/physikalischer Gassensor - wie etwa ein Wärmetönungssensor - oder als physikalischer Gassensor - wie etwa ein Wärmeleitfähigkeitssensor - implementiert sein. Alternativ kann der Gassensor 14 als Feuchtesensor realisiert sein.

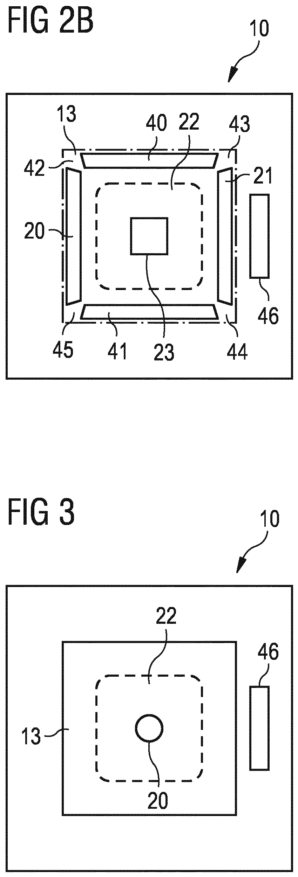

Figur 2B zeigt eine beispielhafte Ausführungsform des Sensorsystems 10 in Aufsicht, die eine Weiterbildung der in Figuren 1 und 2A gezeigten Ausführungsformen des Sensorsystems 10 ist. Die gasdurchlässige Struktur 13 ist als gasdurchlässiges Gitter realisiert und weist zusätzlich zu der im Querschnitt in Figur 2A gezeigten Öffnungen 20, 21 zwei weitere Öffnungen 40, 41 auf. Die Öffnungen 20, 21, 40, 41 sind näherungsweise als Rechtecke oder langgestreckte Schlitze realisiert. Die Öffnungen 20, 21, 40, 41 sind näherungsweise an einer inneren Kante des Rahmens 17 angeordnet. Ausschließlich schmale Stege 42 bis 45 verbinden den Rahmen 17 mit einem inneren Bereich der gasdurchlässigen Struktur 13. Die gasdurchlässigen Struktur 13 kann somit genau vier Öffnungen 20, 21, 40, 41 aufweisen. Die Mitte der gasdurchlässigen Struktur 13 wird beheizt. Somit werden eine Wärmeableitung und damit der Energieverbrauch geringgehalten. Weiter weist der Messbereichs-Halbleiterkörper 15 einen weiteren Temperatursensor 46 auf. Der weitere Temperatursensor 46 ist hier auf dem Rahmen 17 angeordnet. Der weitere Temperatursensor 46 kann zusätzlich oder anstelle des auf der gasdurchlässigen Struktur 13 angeordneten Temperatursensors 23 vorgesehen sein.

Figur 3 zeigt eine weitere beispielhafte Ausführungsform einer Aufsicht auf das Sensorsystem 10, die eine Weiterbildung der in Figuren 1, 2A und 2B gezeigten Ausführungsformen ist. Die gasdurchlässige Struktur 13 weist dabei die Öffnung 20 auf. Die gasdurchlässige Struktur 13 ist als dünne Membran realisiert, so dass mit Vorteil die Heizleistung gering gehalten ist. Die Anzahl der Öffnungen 20 der gasdurchlässigen Struktur 13 kann exakt eins sein. Die Öffnung 20 ist kreisförmig realisiert. Die Öffnung 20 befindet sich näherungsweise in der Mitte der gasdurchlässigen Struktur 13. Die Öffnung 20 befindet sich somit näherungsweise in der Mitte des Rahmens 17.

Alternativ kann die gasdurchlässige Struktur 13 zusätzliche Öffnungen aufweisen. Die zusätzlichen Öffnungen können ebenfalls kreisförmig realisiert sein. Die zusätzlichen Öffnungen können regelmäßig auf der gasdurchlässigen Struktur 13 angeordnet sein. Die gasdurchlässige Struktur 13 ist als Membran realisiert, die die Diffusionsöffnung 16 verschließt. Durch die Wahl der Anzahl der Öffnungen 20 und der Größe der Öffnungen 20 kann die Höhe der Diffusion zwischen einem äußeren Gasraum und dem Messbereich 12 eingestellt werden.

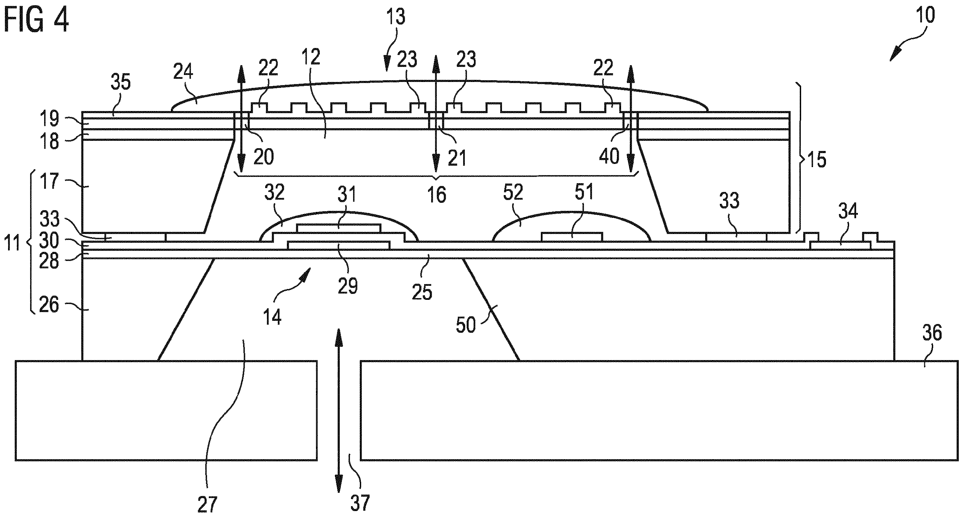

Figur 4 zeigt eine weitere beispielhafte Ausführungsform des Sensorsystems 10, der eine Weiterbildung der in den Figuren 1, 2A, 2B und 3 gezeigten Ausführungsformen ist. Das Sensorsystem 10 umfasst mindestens einen weiteren Sensor 50. Der weitere Sensor 50 und der Gassensor 14 sind auf einem Halbleiterkörper integriert. Der weitere Sensor 50 kann beispielsweise ein weiterer Gassensor oder ein Feuchtesensor sein. Der weitere Sensor 50 kann auf dem Sensorrahmen 26 angeordnet sein. Der weitere Sensor 50 umfasst eine weitere Elektrodenanordnung 51 und eine weitere sensitive Schicht 52. Im Falle eines Feuchtesensors ist die weitere sensitive Schicht 52 ein Feuchte-absorbierendes Dielektrikum. Die weitere Elektrodenanordnung 51 detektiert Änderungen im feuchteempfindlichen Dielektrikum. Die weitere Elektrodenanordnung 51 ist auf der ersten Isolatorschicht 28 angebracht. Das Gas/die Luft im Messbereich 12 ist somit in Kontakt mit dem Gassensor 14 und dem weiteren Sensor 50. Der weitere Sensor 50 detektiert somit einen weiteren Parameter im Messbereich 12.

Der Messbereichs-Halbleiterkörper 12 ist derartig realisiert, dass das mindestens eine Bondpad 34 von außen kontaktiert werden kann. Eine Breite des Messbereichs-Halbleiterkörpers 12 ist somit kleiner als eine Breite des Halbleiterkörpers, der den Gassensor 14 umfasst.

In einer alternativen, nicht gezeigten Ausführungsform ist der weitere Sensor 50 entsprechend dem Gassensor 14 realisiert. Somit umfasst der weitere Sensor 50 eine weitere Sensormembran und eine weitere Ausnehmung.

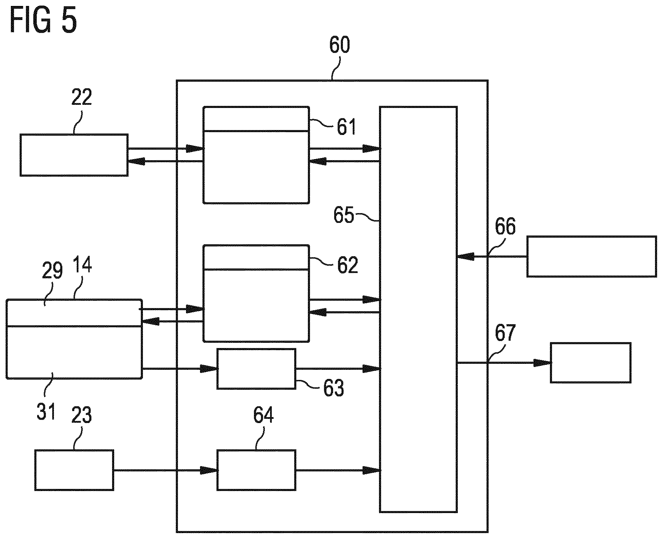

Figur 5 zeigt eine beispielhafte Ausführungsform eines elektrischen Schaltbildes des Sensorsystems 10. Das Sensorsystem 10 umfasst eine Auswerteeinrichtung 60. An die Auswerteeinrichtung 60 sind der Gassensor 14, die Heizeinrichtung 22 und der Temperatursensor 23 angeschlossen. Die Auswerteeinrichtung 60 weist eine Regeleinrichtung 61 auf, die die Heizeinrichtung 22 mit elektrischer Energie in Form einer Heizleistung S1 versorgt. Mittels der Regeleinrichtung 61 ist die Heizeinrichtung 22 regelbar. Die Heizeinrichtung 22 kann als Widerstand realisiert werden. Der Widerstand ist temperaturabhängig.

Der Temperatursensor 23 stellt ein Temperatursensorsignal S2 bereit. Das Temperatursensorsignal S2 kann beispielsweise von einem Widerstandswert des Temperatursensors 23 abhängen.

Die Regeleinrichtung 61 kann unterschiedliche Regelmechanismen aufweisen. Die Regeleinrichtung 61 kann ausgelegt sein, die Heizeinrichtung 22 zuerst einzuschalten und anschließend auszuschalten.

Beispielsweise kann die Regeleinrichtung 61 eine Heizleistung S1 mit einem konstantem Wert SC der Heizeinrichtung 22 in einer ersten Phase A zur Verfügung stellen.

In einer alternativen Ausführungsform kann die Regeleinrichtung 61 ausgelegt sein, eine erste konstante Temperatur des Messbereichs 12 in der ersten Phase A einzustellen. Dabei kann die Regeleinrichtung 61 eine derartige Heizleistung S1 der Heizeinrichtung 22 zur Verfügung stellen, dass ein Widerstandswert der Heizeinrichtung 22 konstant ist. Es kann somit durch die Regeleinrichtung 61 ein konstanter Temperaturwert des Messbereichs 12 eingestellt werden. Der Temperaturwert kann mittels des Temperatursensors 23 bestimmt werden. Die Regeleinrichtung 61 kann ausgelegt sein, eine zweite konstante Temperatur des Messbereichs 12 oberhalb der Raumtemperatur in einer zweiten Phase B einzustellen oder die Heizleistung S1 auf 0 Watt einzustellen und die Heizung somit in der zweiten Phase B abzustellen.

Die Regeleinrichtung 61 kann die Heizleistung S1 pulsweitenmoduliert bereitstellen.

Alternativ kann die Regeleinrichtung 61 derart realisiert sein, dass sie eine Leistungsmodulation durchführt. Beispielsweise kann die Regeleinrichtung 61 stufenweise die Heizleistung S1, die der Heizeinrichtung 22 zur Verfügung gestellt wird, erhöhen und/oder ebenso stufenweise verringern.

Die Auswerteeinrichtung 60 umfasst eine Sensorregeleinrichtung 62, die mit dem Gassensor 14 gekoppelt ist.

Die Sensorheizeinrichtung 29 ist mit der Sensorregeleinrichtung 62 verbunden. Die Sensorregeleinrichtung 62 kann entsprechend einer der oben geschilderten Varianten für die Regeleinrichtung 61 realisiert sein. Dabei kann durch die Sensorregeleinrichtung 62 ein konstanter Temperaturwert der Sensormembran 25 eingestellt werden. Die Heizeinrichtung 22 und die Sensorheizeinrichtung 29 können beispielsweise unabhängig voneinander eingestellt werden.

Der Gassensor 14 stellt ein Sensorsignal S3 bereit. Das Sensorsignal S3 kann beispielsweise als ein Stromsignal ausgebildet sein, wobei eine konstante Spannung an die Elektrodenanordnung 31 angelegt wird. Alternativ kann das Sensorsignal S3 als Spannung ausgebildet sein, wobei ein konstanter Strom über die Elektrodenanordnung 31 durch die sensitive Schicht 32 fließt. Das Sensorsignal S3 kann somit einen Widerstandswert der sensitiven Schicht 32 repräsentieren.

Die Auswerteeinrichtung umfasst einen Mikrocontroller 65, der mit dem Gassensor 14, der Heizeinrichtung 22 und dem Temperatursensor 23 gekoppelt ist. Dabei verbindet die Regeleinrichtung 61 den Mikrocontroller 65 mit der Heizeinrichtung 22. Entsprechend koppelt die Sensorregeleinrichtung 62 den Mikrocontroller 65 mit dem Gassensor 14. Ein Sensorfilter 63 koppelt den Gassensor 14 und den Mikrocontroller 65. Zwischen dem Temperatursensor 23 und dem Mikrocontroller 65 ist ein Temperatursensorfilter 64 angeordnet. Der Mikrocontroller 65 kann mindestens einen Analog-Digital-Wandler zur Digitalisierung des Temperatursensorsignals S2 und/oder des Sensorsignals S3 umfassen. Die Regeleinrichtung 61 und/oder die Sensorregeleinrichtung 62 können auch im Mikrocontroller 65 implementiert sein.

Über einen Eingang 66 werden dem Mikrocontroller 65 Daten zugeleitet. Die Daten können beispielsweise Befehle wie Einschalten und Ausschalten oder Kalibrierdaten sein. Weiter weist der Mikrocontroller 65 mindestens einen Ausgang 67 auf. Der Ausgang 67 kann als Digitalausgang und/oder als Analogausgang realisiert sein. Der Mikrocontroller 65 führt eine Kalkulation unter Verwendung des Sensorsignals S3 des Gassensors 14 durch. Der Mikrocontroller 65 ist ausgelegt, die Heizeinrichtung 22 und die Sensorheizeinrichtung 29 anzusteuern sowie das Sensorsignal S3 und das Temperatursignal S2 auszuwerten. Der Mikrocontroller 65 kann beispielsweise an seinem Ausgang 67 das digitalisierte Sensorsignal S3 bereitstellen.

Alternativ kann der Mikrocontroller 65 an seinem Ausgang 67 eine Information über eine Gaskonzentration bereitstellen, die mittels des Sensorsignals S3 bestimmt ist. Dazu wertet der Mikrocontroller 61 Werte des Sensorsignals S3, die während oder am Ende einer Heizphase bestimmt worden sind, sowie Werte des Sensorsignals S3, die nach dem Abschalten der Heizung oder dem Verringern der Heizleistung S1 auf eine von 0 Watt verschiedenen Wert bestimmt worden sind, aus. Alternativ kann anstelle des Mikrocontrollers 65 ein Mikroprozessor vorgesehen sein.

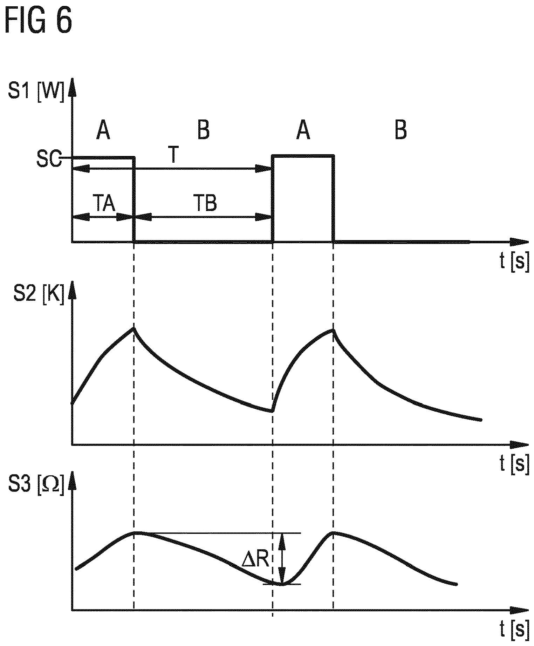

Figur 6 zeigt eine beispielhafte Ausführungsform der Signale im Sensorsystem 10. Die Heizleistung S1, das Temperatursensorsignal S2 sowie das Sensorsignal S3 sind über der Zeit t aufgetragen. Eine Periode besteht aus der ersten und der zweiten Phase A, B. Während in der ersten Phase A die Heizleistung S1 den Wert SC annimmt, hat die Heizleistung S1 in der zweiten Phase B den Wert 0. Eine Periodendauer T der Periode ist somit die Summe aus einer ersten Dauer TA der ersten Phase und einer zweiten Dauer TB der zweiten Phase B. Die erste Phase A und die zweite Phase B alternieren. Die Periodendauer T kann konstant sein. Das Temperatursensorsignal S2 steigt in der ersten Phase A an und fällt in der zweiten Phase B ab. Die zweite Dauer TB der zweiten Phase B kann so kurz gewählt sein, dass das Temperatursensorsignal S2 nicht auf den Wert bei Raumtemperatur abfällt.

Gemäß der allgemeinen Gasgleichung gilt:

$$p \cdot V = n \cdot R_m \cdot T \, ,$$

wobei p der Druck im Messbereich 12, V das Volumen des Messbereichs 12, n die Stoffmenge aller Gase im Messbereich 12, $R_m$ die allgemeine Gaskonstante und T die absolute Temperatur im Messbereich 12 ist. Der Druck p und das Volumen V des Messbereichs 12 sind näherungsweise konstant. Daher ist die Stoffmenge und somit die Teilchenzahl aller Gase und damit die Konzentration eines Gases proportional zum Kehrwert 1/T der absoluten Temperatur. Bei hoher Temperatur im Messbereich 12 ist somit die Konzentration der Moleküle geringer als bei niedriger Temperatur im Messbereich 12. Aus obiger Gleichung ergibt sich die folgende Gleichung:

$$n_2 = n_1 \cdot T_1 / T_2 \, ,$$

 wobei $n_2$ die Stoffmenge im Messbereich 12 bei der Temperatur $T_2$ im Messbereich 12 und $n_1$ die Stoffmenge im Messbereich 12 bei der Temperatur $T_1$ im Messbereich 12 ist.

Die Werte für die erste Dauer TA, die zweite Dauer TB und die Heizleistung S1 in der ersten Phase A werden derart gewählt, dass eine Konzentrationsänderung des Gases im Messbereich 12 entsteht. Ein Abschalten der Heizungseinrichtung 22 beim Wechsel von der ersten Phase A zur zweiten Phase B führt somit zu einer Zunahme der Konzentration des Gases.

Das Sensorsignal S3 hängt von der Konzentration des Gases ab, für das die sensitive Schicht 32 empfindlich ist, und hängt deshalb vom Temperatursensorsignal S2 ab. Das Sensorsignal S3 kann der Widerstandswert des Gassensors 14 sein. Bei einer sensitiven Schicht 32 aus $SnO_2$ führt die Zunahme beispielsweise einer Kohlenmonoxid-Konzentration im Messbereich 12 zu einer Verringerung des Sensorsignals S3. Damit ergibt sich eine Widerstandsänderung $\Delta R$. Zur Bestimmung der Konzentration des Gases werden mindestens ein Wert des Sensorsignals S3 in der ersten Phase A und mindestens ein Wert des Sensorsignals S3 in der zweiten Phase B gemessen. Die Konzentration des Gases kann aus den so ermittelten Werten des Sensorsignals S3 bestimmt werden. Die durch das Sensorsystem 10 bestimmte Konzentration des Gases ist eine Funktion des mindestens einen Werts des Sensorsignals S3 in der ersten Phase A und des mindestens einen Werts des Sensorsignals S3 in der zweiten Phase B. Beispielsweise kann die durch das Sensorsystem 10 bestimmte Konzentration eine Funktion der Differenz $\Delta R$ der Werte des Sensorsignals S3 sein.

Die Temperatur des Gassensors 14 wird in einer Periode näherungsweise konstant gehalten. Zur Erhöhung der Selektivität kann die Temperatur des Gassensors 14 in aufeinanderfolgenden Perioden unterschiedlich eingestellt werden.

In einer alternativen, nicht gezeigten Ausführungsform weist ein Signalverlauf mehr als zwei Phasen auf. Die mehr als zwei Phasen weisen unterschiedliche Heizleistungen auf.

Alternativ hat die Heizleistung S1 während der zweiten Phase B einen Wert größer 0 Watt.

**Patentansprüche**

1. Verfahren zur Messung der Konzentration mindestens eines Gases aus einer Gasprobe mit einem Sensorsystem (10), das einen Messbereich (12) mit mindestens einem Gassensor (14) aufweist, wobei der Messbereich (12) eine Diffusionsöffnung (16) aufweist, die durch eine gasdurchlässige Struktur (13) verschlossen ist, **dadurch gekennzeichnet,** dass der Messbereich (12) zunächst aufgeheizt wird, dann eine Heizung abgeschaltet oder die Heizleistung (S1) auf einen von 0 verschiedenen Wert verringert wird und die Widerstandsänderung des mindestens einen Gassensors (14) gemessen wird.

2. Verfahren nach Anspruch 1, **dadurch gekennzeichnet, dass** die Selektivität des Sensorsystems (10) durch eine Sensorheizeinrichtung (29) für den mindestens einen Gassensor (14) und/oder durch die Heizung des Messbereichs (12) eingestellt wird.

3. Verfahren nach Anspruch 1 oder 2, **dadurch gekennzeichnet, dass** die Gasprobe ein Einzelgas, ein Gasgemisch und/oder ein Aerosol ist.

4. Verfahren nach einem der vorhergehenden Ansprüche, **dadurch gekennzeichnet, dass** die Gasprobe durch mindestens eine Katalysatoranordnung hindurchströmt und/oder diffundiert, bevor sie das Sensorsystem (10) oder den Messbereich (12) erreicht.

5. Verfahren nach einem der Ansprüche 1 bis 4, wobei der Gassensor (14) als ein Metall-Oxid-Sensor realisiert ist.

6. Verfahren nach einem der Ansprüche 1 bis 5, wobei das Sensorsystem (10) eine Heizeinrichtung (22) zum Aufheizen der Gasprobe im Messbereich (12) umfasst.

7. Verfahren nach einem der Ansprüche 1 bis 6, wobei der mindestens eine Gassensor (14) eine Sensorheizeinrichtung (29) zum Aufheizen einer sensitiven Schicht (32) des Gassensors (14) umfasst.

8. Verfahren nach einem der Ansprüche 1 bis 7, wobei die Konzentration eines Gases der Gasprobe im Messbereich (12) in einer ersten Phase (A), während der der Messbereich (12) aufgeheizt wird, verschieden von der Konzentration des Gases der Gasprobe im Messbereich (12) in einer zweiten Phase (B) ist, während der die Heizung abgeschaltet oder die Heizleistung auf den von 0 verschiedenen Wert verringert ist.

9. Sensorsystem zur Messung der Konzentration mindestens eines Gases aus einer Gasprobe mit einem Messbereich (12), in dem mindestens ein Gassensor (14) angeordnet ist und der eine Diffusionsöffnung (16) aufweist, die durch eine gasdurchlässige Struktur (13) verschlossen ist, wobei der Messbereich (12) mit einer regelbaren Heizeinrichtung (22) für den Messbereich versehen ist, **dadurch gekennzeichnet,**

**dass** das Sensorsystem (10) derart eingerichtet ist, dass der Messbereich (12) zunächst durch die Heizeinrichtung (22) aufgeheizt wird und dann die Heizung abgeschaltet oder die Heizleistung (S1) auf einen von 0 verschiedenen Wert verringert wird sowie die Widerstandsänderung des mindestens einen Gassensors (14) gemessen wird.

10. Sensorsystem nach Anspruch 9, **dadurch gekennzeichnet, dass** die die Diffusionsöffnung (16) des Messbereichs verschließende gasdurchlässige Struktur (13) durch die Heizeinrichtung (22) beheizbar ist.

11. Sensorsystem nach Anspruch 9 oder 10, **dadurch gekennzeichnet, dass** der mindestens eine Gassensor (14) durch eine Sensorheizeinrichtung (29) beheizbar ist.

12. Sensorsystem nach einem der Ansprüche 9 bis 11, **dadurch gekennzeichnet, dass** die die Diffusionsöffnung (16) des Messbereichs (12) verschließende gasdurchlässige Struktur (13) ein gasdurchlässiges Gitter, ein Netz, ein poröser Festkörper, ein Schwamm oder eine Membran ist.

13. Sensorsystem nach einem der Ansprüche 9 bis 12, wobei die gasdurchlässige Struktur (13) eine gaspermeable Abdeckschicht (24) umfasst.

14. Sensorsystem nach einem der Ansprüche 9 bis 13, wobei der Gassensor (14) als ein Metall-Oxid-Sensor realisiert ist.

15. Sensorsystem nach einem der Ansprüche 9 bis 14, wobei das Sensorsystem (10) derart eingerichtet ist, dass die Konzentration eines Gases der Gasprobe im Messbereich (12) in einer ersten Phase (A), während der der Messbereich (12) aufgeheizt wird, verschieden von der Konzentration des Gases der Gasprobe im Messbereich (12) in einer zweiten Phase (B) ist, während der die Heizung abgeschaltet oder die Heizleistung auf den von 0 verschiedenen Wert verringert ist.

**Claims**

1. Method for measuring the concentration of at least one gas from a gas sample by a sensor system (10) which has a measuring area (12) with at least one gas sensor (14), the measuring area (12) having a diffusion opening (16) which is closed by a gas-permeable structure (13),
   **characterized in**
   **that** the measuring area (12) is initially heated up, a heating is then switched off or the heating power is reduced to a value different from 0 and the change in resistance of the at least one gas sensor (14) is measured.

2. Method according to claim 1, **characterized in that** the selectivity of the sensor system (10) is set by a sensor heating device (29) for the at least one gas sensor (14) and/or by the heating of the measuring area (12).

3. Method according to claim 1 or 2, **characterized in that** the gas sample is an individual gas, a gas mixture and/or an aerosol.

4. Method according to one of the preceding claims,
   **characterized in that** the gas sample flows and/or diffuses through at least one catalyst arrangement before it reaches the sensor system (10) or the measuring area (12).

5. Method according to one of claims 1 to 4, wherein the gas sensor (14) is implemented as a metal oxide sensor.

6. Method according to one of claims 1 to 5, wherein the sensor system (10) comprises a heating device (22) for heating the gas sample in the measuring area (12).

7. Method according to one of claims 1 to 6, wherein the at least one gas sensor (14) comprises a sensor heating device (29) for heating a sensitive layer (32) of the gas sensor (14).

8. Method according to one of claims 1 to 7, wherein the concentration of a gas from the gas sample in the measuring area (12) in a first phase (A) during which the measuring area (12) is heated up is different from the concentration of the gas from the gas sample in the measuring area (12) in a second phase (B), during which the heating is switched off or the heating power is reduced to the value different from 0.

**9.** Sensor system for measuring the concentration of at least one gas from a gas sample having a measuring area (12) in which at least one gas sensor (14) is arranged and which has a diffusion opening (16) which is closed by a gas-permeable structure (13), wherein the measuring area (12) is provided with a controllable heating device (22) for the measuring area,

**characterized in**

**that** the sensor system (10) is configured such that the measuring area (12) is initially heated up by the heating device (22) and then the heating is switched off or the heating power (S1) is reduced to a value different from 0 and the change in resistance of the at least one gas sensor (14) is measured.

**10.** Sensor system according to claim 9, **characterized in that** the gas-permeable structure (13) closing the diffusion opening (16) of the measuring area is heatable by the heating device (22).

**11.** Sensor system according to claim 9 or 10, **characterized in that** the at least one gas sensor (14) is heatable by a sensor heating device (29).

**12.** Sensor system according to one of claims 9 to 11, **characterized in that** the gas-permeable structure (13) closing the diffusion opening (16) of the measuring area (12) is a gas-permeable grid, a mesh, a porous solid body, a sponge or a membrane.

**13.** Sensor system according to one of claims 9 to 12, wherein the gas-permeable structure (13) comprises a gas-permeable covering layer (24).

**14.** Sensor system according to one of claims 9 to 13, wherein the gas sensor (14) is implemented as a metal oxide sensor.

**15.** Sensor system according to one of claims 9 to 14, wherein the sensor system (10) is configured such that the concentration of a gas from the gas sample in the measuring area (12) in a first phase (A), during which the measuring area (12) is heated up, is different from the concentration of the gas from the gas sample in the measuring area (12) in a second phase (B), during which the heating is switched off or the heating power is reduced to the value different from 0.

**Revendications**

**1.** Procédé pour la mesure de la concentration d'au moins un gaz issu d'un échantillon de gaz avec un système de capteur (10) qui présente une zone de mesure (12) comprenant au moins un capteur de gaz (14), sachant que la zone de mesure (12) présente une ouverture de diffusion (16) qui est obturée par une structure (13) perméable aux gaz,

**caractérisé en ce que**

la zone de mesure (12) est d'abord réchauffée, puis un chauffage est coupé ou la puissance de chauffe (S1) est réduite à une valeur différente de 0 et la variation de résistance de l'au moins un capteur de gaz (14) est mesurée.

**2.** Procédé selon la revendication 1, **caractérisé en ce que** la sélectivité du système de capteur (10) est réglée par un dispositif de chauffe de capteur (29) pour l'au moins un capteur de gaz (14) et/ou par le chauffage de la zone de mesure (12).

**3.** Procédé selon la revendication 1 ou 2, **caractérisé en ce que** l'échantillon de gaz est un gaz individuel, un mélange de gaz et/ou un aérosol.

**4.** Procédé selon l'une des revendications précédentes, **caractérisé en ce que** l'échantillon de gaz s'écoule et/ou se diffuse à travers au moins un agencement de catalyseur avant d'atteindre le système de capteur (10) ou la zone de mesure (12).

**5.** Procédé selon l'une des revendications 1 à 4, sachant que le capteur de gaz (14) est réalisé comme capteur à oxyde métallique.

**6.** Procédé selon l'une des revendications 1 à 5, sachant que le système de capteur (10) comprend un dispositif de chauffe (22) pour le réchauffage de l'échantillon de gaz dans la zone de mesure (12).

**7.** Procédé selon l'une des revendications 1 à 6, sachant que l'au moins un capteur de gaz (14) comprend un dispositif de chauffe de capteur (29) pour le réchauffage d'une couche sensible (32) du capteur de gaz (14) .

**8.** Procédé selon l'une des revendications 1 à 7, sachant que la concentration d'un gaz de l'échantillon de gaz dans la zone de mesure (12) dans une première phase (A) pendant laquelle la zone de mesure (12) est réchauffée est différente de la concentration du gaz de l'échantillon de gaz dans la zone de mesure (12) dans une deuxième phase (B) pendant laquelle le chauffage est coupé ou la puissance de chauffe est réduite à la valeur différente de 0.

**9.** Système de capteur pour la mesure de la concentration d'au moins un gaz issu d'un échantillon de gaz avec une zone de mesure (12) dans laquelle au moins un capteur de gaz (14) est disposé et qui présente une ouverture de diffusion (16) qui est obturée par une structure (13) perméable aux gaz, sachant que la zone de mesure (12) est pourvue d'un dispositif de chauffe (22) réglable pour la zone de mesure,
**caractérisé en ce que**
le système de capteur (10) est configuré de telle façon que la zone de mesure (12) soit d'abord réchauffée par le dispositif de chauffe (22) puis le chauffage soit coupé ou la puissance de chauffe (S1) soit réduite à une valeur différente de 0 ainsi que la variation de résistance de l'au moins un capteur de gaz (14) soit mesurée.

**10.** Système de capteur selon la revendication 9, **caractérisé en ce que** la structure (13) perméable aux gaz obturant l'ouverture de diffusion (16) de la zone de mesure peut être chauffée par le dispositif de chauffe (22).

**11.** Système de capteur selon la revendication 9 ou 10, **caractérisé en ce que** l'au moins un capteur de gaz (14) peut être chauffé par un dispositif de chauffe de capteur (29).

**12.** Système de capteur selon l'une des revendications 9 à 11, **caractérisé en ce que** la structure (13) perméable aux gaz obturant l'ouverture de diffusion (16) de la zone de mesure (12) est une grille perméable aux gaz, un filet, un corps solide poreux, une éponge ou une membrane.

**13.** Système de capteur selon l'une des revendications 9 à 12, sachant que la structure (13) perméable aux gaz comprend une couche de recouvrement (24) perméable aux gaz.

**14.** Système de capteur selon l'une des revendications 9 à 13, **caractérisé en ce que** le capteur de gaz (14) est réalisé comme capteur à oxyde métallique.

**15.** Système de capteur selon l'une des revendications 9 à 14, sachant que le système de capteur (10) est configuré de telle façon que la concentration d'un gaz de l'échantillon de gaz dans la zone de mesure (12) dans une première phase (A) pendant laquelle la zone de mesure (12) est réchauffée est différente de la concentration du gaz de l'échantillon de gaz dans la zone de mesure (12) dans une deuxième phase (B) pendant laquelle le chauffage est coupé ou la puissance de chauffe est réduite à la valeur différente de 0.

## FIG 1

FIG 2A

## FIG 2B

10

13 40 22 43

42 21

20 20

45 41 23 44 46

## FIG 3

10

22

46

13

20

FIG 4

EP 3 105 571 B1

FIG 5

# FIG 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20090084160 A1

- JP 2005134311 A

**In der Beschreibung aufgeführte Nicht-Patentliteratur**

- **R. E. CAVICCHI et al.** Fast Temperature Programmed Sensing for Micro-Hotplate Gas Sensors. *IEEE Electron Device Letters,* Juni 1995, vol. 16 (6), 286-288